(19) Europäisches Patentamt
European Patent Office
Office européen des brevets

(11) Veröffentlichungsnummer: **0 218 068 A1**

(12) # EUROPÄISCHE PATENTANMELDUNG

(21) Anmeldenummer: **86111731.5**

(22) Anmeldetag: **25.08.86**

(51) Int. Cl.⁴: **C 07 D 211/90**, C 07 D 413/04, C 07 D 401/12, C 07 D 401/04, C 07 D 405/04, C 07 D 405/12, C 07 D 409/04, C 07 D 409/12, C 07 D 413/12, C 07 D 417/04, C 07 D 417/12

(30) Priorität: **04.09.85 DE 3531498**

(43) Veröffentlichungstag der Anmeldung: **15.04.87 Patentblatt 87/16**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Schwenner, Eckhard, Dr., Claudiusweg 3, D-5600 Wuppertal 1 (DE)**
Erfinder: **Kinast, Günther, Dr., Am Eckbusch 15a, D-5600 Wuppertal 1 (DE)**
Erfinder: **Knorr, Andreas, Dr., Pahlkestrasse 15, D-5600 Wuppertal 1 (DE)**
Erfinder: **Kazda, Stanislav, Dr., Gellertweg 18, D-5600 Wuppertal 1 (DE)**

(54) **Dihydropyridin-2-hydroxyamine, Verfahren zur Herstellung und ihre Verwendung in Arzneimitteln.**

(57) Dihydropyridin-2-hydroxy-amine der allgemeinen Formel (I)

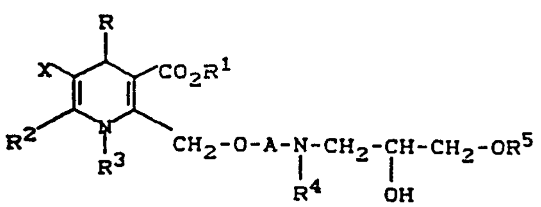

(I)

in welcher
für gegebenenfalls substituiertes Aryl oder Heteroaryl, z.B. 2-Chlorphenyl oder 4-Benzoxadiazolyl, steht,
$R^1$ einen geradkettigen, verzweigten oder cyclischen, gesättigten oder ungesättigten Kohlenwasserstoffrest bedeutet, der gegebenenfalls durch ein Sauerstoff- oder ein Schwefelatom in der Kette unterbrochen ist, und/oder der gegebenenfalls substituiert ist.
$R^2$ für Wasserstoff, Cyano, Aryl, Aralkyl oder für geradkettiges oder verzweigtes Alkyl steht, das gegebenenfalls substituiert ist durch Cyano, OH, Acetoxy oder Halogen,
$R^3$ für Wasserstoff oder geradkettiges oder verzweigtes Alkyl steht, das gegebenenfalls durch ein oder zwei Sauerstoffatome in der Alkylkette unterbrochen ist, oder für Aryl oder Aralkyl steht,
$R^4$ für Wasserstoff, geradkettiges oder verzweigtes Alkyl, Aryl, Aralkyl oder für einen Rest der Formel

$$CO\text{-}R^6,\ CO_2\text{-}R^{6'},\ CO\text{-}NR^6R^7,\ SO_2R^6,\ \text{-}CH_2\text{-}\underset{\displaystyle OH}{\underset{|}{CH}}\text{-}CH_2\text{-}OR^8$$

steht,
$R^5$ für gegebenenfalls substituiertes Aryl oder Heteroaryl, z.B. 1-Naphtyl oder 4-Indolyl, steht,
A für eine geradkettige, verzweigte oder cyclische Alkylengruppe mit bis zu 20 Kohlenstoffatomen steht, die durch eine gegebenenfalls durch Halogen, Cyano, Dialkylamino, Alkyl, Alkoxy, Trifluormethyl oder Nitro substituierte Phenylgruppe substituiert oder unterbrochen sein kann, und/oder die gegebenenfalls durch ein oder mehrere Sauerstoffatome, Schwefelatome oder Gruppierungen der Formel $-NR^6-$ oder $-CO-NR^7$ unterbrochen ist, wobei
X für die Gruppe $-COR^9$ oder für die Gruppe $-COOR^{10}$ steht,
sowie deren physiologisch unbedenklichen Säureadditionsprodukte, Verfahren zu ihrer Herstellung, Zwischenprodukte zu ihrer Herstellung sowie ihre Verwendung in Arzneimitteln, insbesondere in kreislaufbeeinflussenden Arzneimitteln.

EP 0 218 068 A1

BAYER AKTIENGESELLSCHAFT 5090 Leverkusen, Bayerwerk
Konzernverwaltung RP
Patentabteilung KS/ABc

# Dihydropyridin-2-hydroxyamine, Verfahren zu ihrer Herstellung und ihre Verwendung in Arzneimitteln

Die vorliegende Erfindung betrifft neue Dihydropyridin-2-hydroxyamine, Verfahren zu ihrer Herstellung, Zwischenprodukte zu ihrer Herstellung sowie ihre Verwendung in Arzneimitteln, insbesondere in kreislaufbeeinflussenden Arzneimitteln.

Es ist bereits bekannt worden, daß man 1,4-Dihydro-2,6-dimethyl-4-phenyl-pyridin-3,5-dicarbonsäurediethylester erhält, wenn man 2-Benzylidenacetessigsäureethylester mit β-Aminocrotonsäureethylester oder Acetessigsäureethylester und Ammoniak umsetzt (E. Knoevenagel, Ber. dtsch. chem. Ges. 31, 743 (1898)).

Weiterhin ist bekannt, daß bestimmte 1,4-Dihydropyridine interessante pharmakologische Eigenschaften aufweisen (F. Bossert, W. Vater, Naturwissenschaften 58, 578 (1971).

Ebenso ist es aus den deutschen Offenlegungsschriften 28 47 236 und 22 18 644 bekannt, daß Dihydropyridinester als Coronarmittel und blutdrucksenkende Mittel verwendet werden können.

Die vorliegende Erfindung betrifft 2-substituierte 1,4-Dihydropyridinderivate der allgemeinen Formel (I)

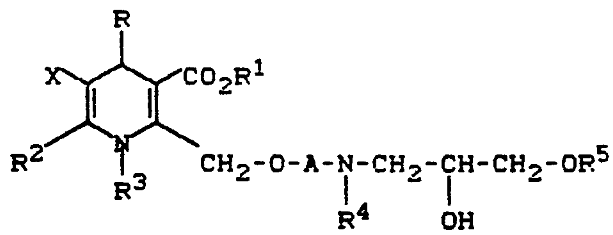

(I)

in welcher

R für Aryl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei der Arylrest sowie die Heterocyclen gegebenenfalls 1 bis 2 gleiche oder verschiedene Substituenten aus der Gruppe Phenyl, Alkyl, Alkoxy, Alkylen, Dioxyalkylen, Halogen, Trifluormethyl, Polyfluoralkoxy, Nitro, Cyano, Azido oder $SO_m$-Alkyl (m = 0 bis 3) enthalten,

$R^1$ einen geradkettigen, verzweigten oder cyclischen, gesättigten oder ungesättigten Kohlenwasserstoffrest bedeutet, der gegebenenfalls durch ein Sauerstoff- oder ein Schwefelatom in der Kette unterbrochen ist,

und/oder der gegebenenfalls substituiert ist durch Halogen, Cyan, Hydroxy, Acyloxy, Pyridyl oder durch eine Phenyl-, Phenoxy-, Phenylthio- oder Phenylsulfonylgruppe, wobei die Arylreste ihrerseits wieder durch Halogen, Cyano, Dialkylamino, Alkoxy, Alkyl, Trifluormethyl oder Nitro substituiert sein können,

oder der gegebenenfalls durch eine Aminogruppe substituiert ist, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem Stickstoffatom einen 5- bis 7-gliedrigen Ring bilden, der als weiteres Heteroatom ein Sauerstoff- oder Schwefelatom oder eine N-Alkyl-Gruppierung enthalten kann,

$R^2$ für Wasserstoff, Cyano, Aryl, Aralkyl oder für geradkettiges oder verzweigtes Alkyl steht, das gegebenenfalls substituiert ist durch Cyano, OH, Acetoxy oder Halogen,

$R^3$ für Wasserstoff oder geradkettiges oder verzweigtes Alkyl steht, das gegebenenfalls durch ein oder zwei Sauerstoffatome in der Alkylkette unterbrochen ist, oder für Aryl oder Aralkyl steht,

$R^4$ für Wasserstoff,
für geradkettiges oder verzweigtes Alkyl,
für Aryl, Aralkyl oder
für einen Rest der Formel $CO-R^6$, $CO_2-R^{6'}$, $CO-NR^6R^7$, $SO_2R^6$, $-CH_2-\underset{\displaystyle OH}{\underset{|}{CH}}-CH_2-OR^8$ steht,

wobei

$R^6$ und $R^7$ gleich oder verschieden sind und jeweils für Wasserstoff, einen niedrigen Kohlenwasserstoffrest, Aryl oder Aralkyl stehen,

$R^{6'}$ die Bedeutung von $R^6$ hat, jedoch nicht Wasserstoff bedeutet,

$R^8$ die für $R^5$ angegebene Bedeutung hat und mit diesem gleich oder verschieden sein kann,

$R^5$ für Aryl oder Heteroaryl steht, wobei die Aryle sowie die Heteroaryle gegebenenfalls 1 bis 3 gleiche oder verschiedene Substituenten aus der Gruppe Phenyl, Alkyl, Cycloalkyl, Morpholinyl, Alkoxy, Alkylen, Alkenyl, Alkenyloxy, Dioxyalkylen, Halogen, Polyfluoralkoxy, Nitro, Cyano, Azido, $SO_m-R^6$, $-R^6R^7N-SO_m-$ ($m = 0$ bis 2), $NR^6R^7$, $NR^6COR^7$ und Acyl der Formel $CO-R^6$ enthalten können, und wobei die Alkylreste, ihrerseits gegebenenfalls durch ein oder zwei Sauerstoffatome, Schwefelatome oder

Gruppierungen der Formel $-NR^6-$, $-CONR^7-$ oder $-\underset{H}{N}CO_2-$ unterbrochen sein kann und/oder durch Halogen substituiert sein kann, und wobei $R^6$ und $R^7$ jeweils gleich oder verschieden sind und die obengenannte Bedeutung haben,

A für eine geradkettige, verzweigte oder cyclische Alkylengruppe mit bis zu 20 Kohlenstoffatomen steht, die durch eine gegebenenfalls durch Halogen, Cyano, Dialkylamino, Alkyl, Alkoxy, Trifluormethyl oder Nitro substituierte Phenylgruppe substituiert oder unterbrochen sein kann, und/oder die gegebenenfalls durch ein oder mehrere Sauerstoffatome, Schwefelatome oder Gruppierungen der Formel $-NR^6-$ oder $-CO-NR^7-$ unterbrochen ist, wobei

$R^6$, $R^7$ die oben angegebene Bedeutung haben,

und

X für die Gruppe $-COR^9$ steht, worin $R^9$ gegebenenfalls substituiertes Alkyl, Aryl, Aralkyl oder Anilino oder eine Amino-, Monoalkylamino- oder Dialkylaminogruppe bedeutet, wobei gegebenenfalls die Alkylgruppen mit dem Stickstoffatom einen 5- bis 7-gliedrigen Ring bilden, der als weiteres Heteroatom ein Sauerstoff- oder Schwefelatom enthalten kann,

oder für die Gruppe $-COOR^{10}$ steht, worin $R^{10}$ die gleiche Bedeutung hat wie $R^1$ und mit diesem gleich oder verschieden sein kann,

sowie deren physiologisch unbedenklichen Säureadditionsprodukte.

Die Herstellung der erfindungsgemäßen Verbindungen der allgemeinen Formel (I) erfolgt dadurch, daß man Dihydropyridine der allgemeinen Formel (II)

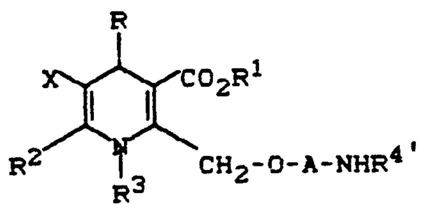

(II)

in welcher

$R$, $R^1$, $R^2$, $R^3$, X und A die oben angegebene Bedeutung haben, und

$R^{4'}$ für Wasserstoff, Alkyl, Aryl oder Aralkyl steht,

mit Epoxiden der allgemeinen Formel (III)

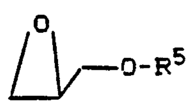

(III)

in welcher

$R^5$ die oben angegebene Bedeutung hat,

gegebenenfalls in Gegenwart inerter organischer Lösungsmittel umsetzt.

Die Herstellung von Verbindungen der allgemeinen Formel (I), in welcher $R^4$ für den Rest $CH_2-\underset{OH}{\underset{|}{CH}}-CH_2-OR^8$ steht,

wobei $R^8$ von $R^5$ verschieden ist, erfolgt vorzugsweise durch stufenweise Umsetzung von primären Dihydropyridinaminen (II) mit zunächst einem Epoxid mit dem Substituenten $R^5$ (III) und anschließend einem weiteren Epoxid mit dem Substituenten $R^8$ der allgemeinen Formel IIIa

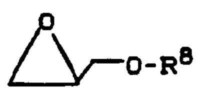

(IIIa).

Die Herstellung von Verbindungen der allgemeinen Formel (I), in welcher $R^4$ für die Reste CO-$R^6$ $CO_2R^{6'}$, CO-$NR^6R^7$ oder $SO_2R^{6'}$ steht, erfolgt durch Umsetzung der Verbindungen (I), in welchen $R^4$ Wasserstoff ist, mit Acylierungs- oder Sulfonylierungsmitteln wie $R^{6'}CO_2H$, $R^{6'}$-COCl, $(R^{6'}CO)_2O$, $R^{6'}O_2CCl$, $R^6R^7N$-COCl, $R^6$-NCO, $R^{6'}$-$SO_2Cl$, in welchen $R^6$, $R^{6'}$, $R^7$ die oben angegebene Bedeutung haben.

Das erfindungsgemäße Verfahren kann durch die in den folgenden Formelschemata angeführten Beispiele verdeutlicht werden.

a)

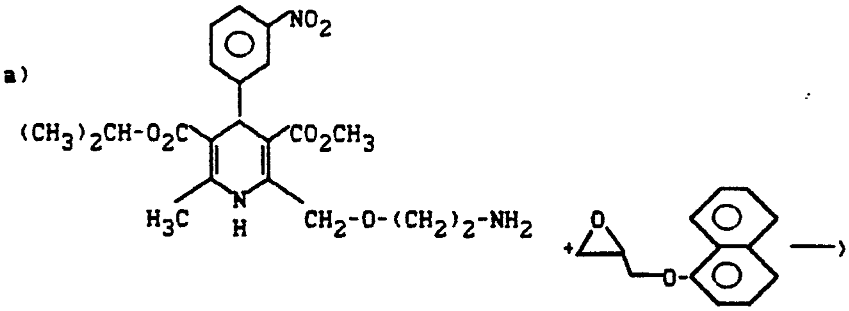

b)

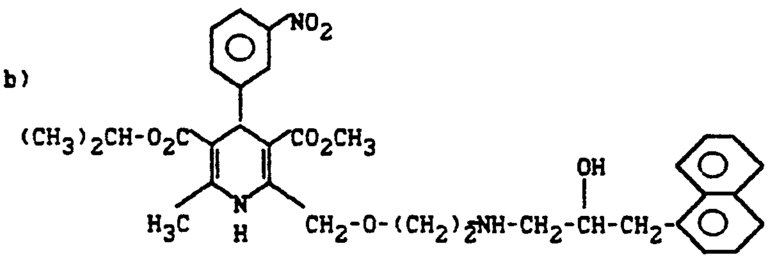

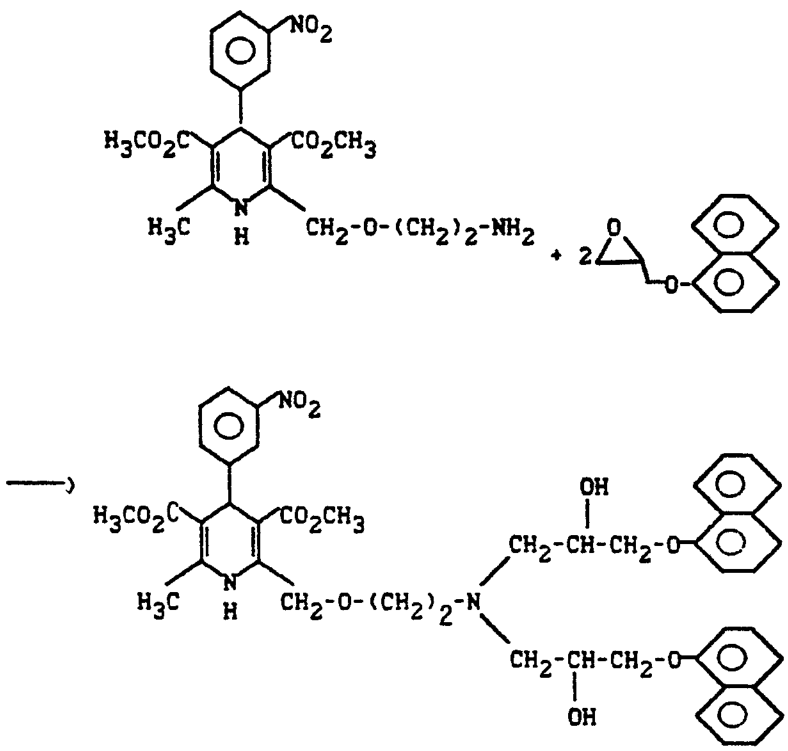

c)

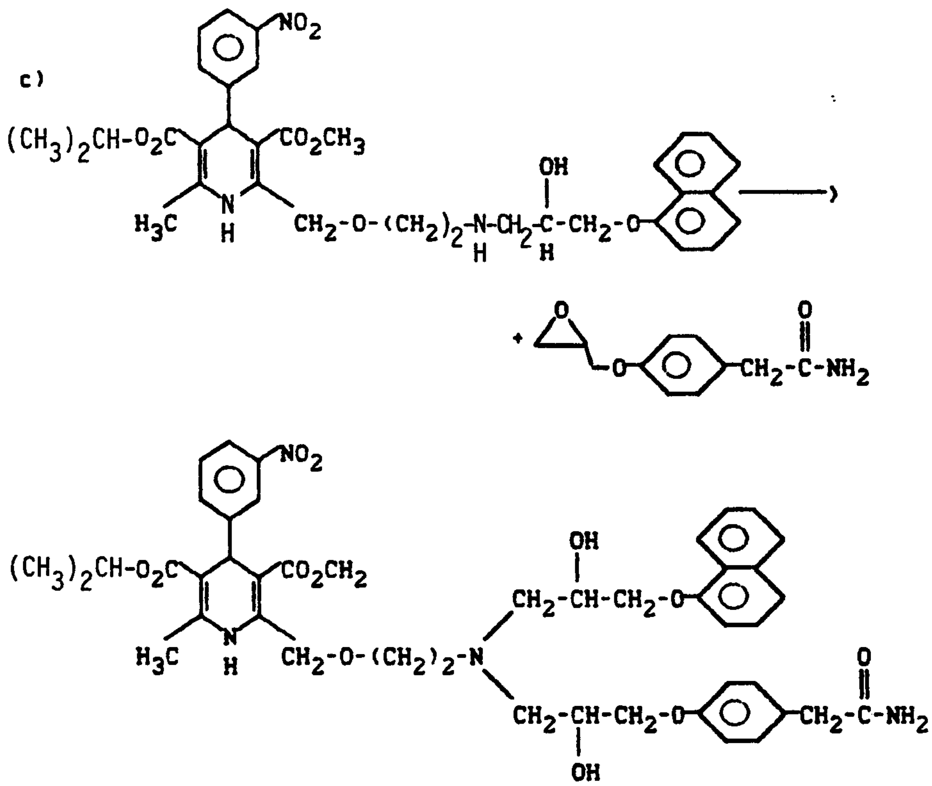

d)

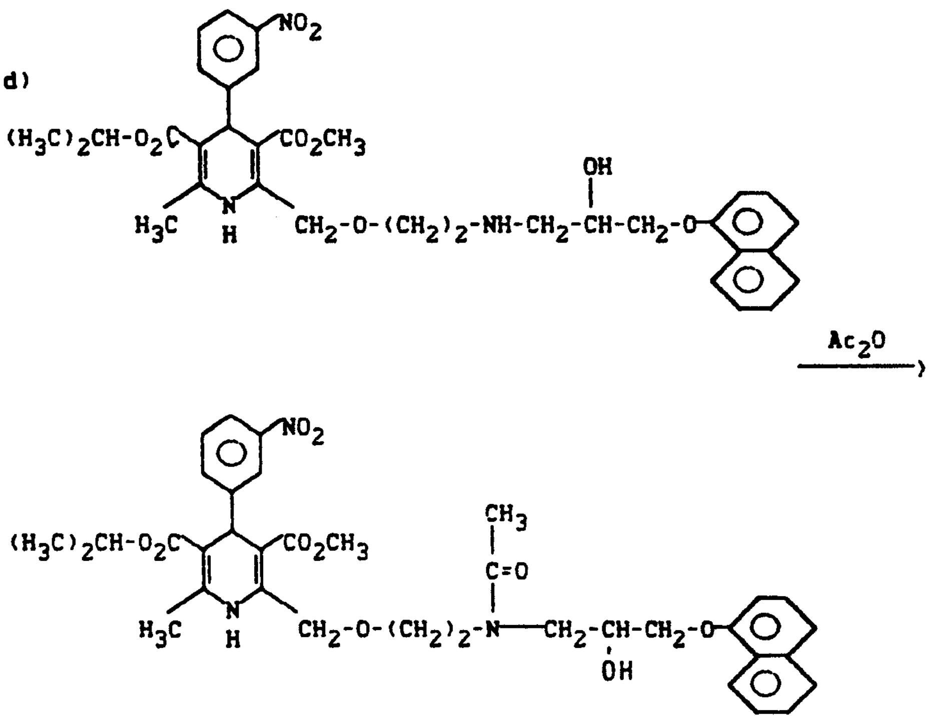

Von besonderem Interesse sind die erfindungsgemäßen Verbindungen der allgemeinen Formel (I)

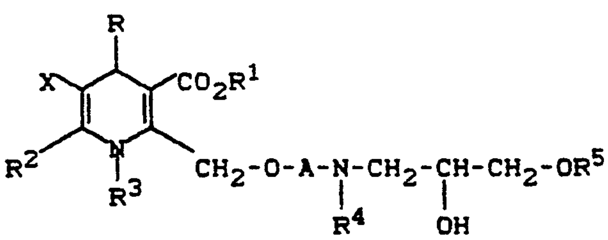

(I)

in welcher

R für Phenyl, Naphthyl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit bis zu 8 Kohlenstoffatomen, Cycloalkyl mit 3 bis 7 Kohlenstoffatomen, Alkoxy mit 1 bis 4 Kohlenstoffatomen, Tri-, Tetra- und Pentamethylen, Dioxymethylen, Halogen, Trifluormethyl, Trifluormethoxy, Difluormethoxy, Tetrafluorethoxy, Nitro, Cyano, Azido oder $SO_m$-Alkyl, worin m eine Zahl von 0 bis 2 bedeutet und Alkyl vorzugsweise 1 bis 4 Kohlenstoffatomen enthält, substituiert sein können,

$R^1$ einen geradkettigen, verzweigten oder cyclischen, gesättigten oder ungesättigten Kohlenwasserstoffrest mit bis zu 16 C-Atomen bedeutet, der gegebenenfalls durch ein Sauerstoff- oder ein Schwefelatom in der Kette unterbrochen ist, und/oder der gegebenenfalls substituiert ist durch Fluor, Chlor, Brom, Cyano, Hydroxy, Pyridyl, Acyloxy mit bis zu 4 C-Atomen oder durch eine Phenyl-, Phenoxy-, Phenylthio- oder Phenylsulfonylgruppe, welche ihrerseits wieder durch Fluor, Chlor, Brom, Cyano, Dialkylamino (1 bis 4 C-Atome), Alkoxy (1 bis 4 C-Atome), Alkyl (1 bis 4 C-Atome), Trifluormethyl oder Nitro substituiert sein können, oder der gegebenenfalls durch eine Aminogruppe substituiert ist, wobei diese Aminogruppe zwei gleiche oder verschiedene Substituenten aus der Gruppe Alkyl (1 bis 4 C-Atome), Alkoxyalkyl (bis zu 8 C-Atome), Phenyl, Naphthyl oder Phenylalkyl mit 1 bis 4 C-Atomen im Alkylrest trägt und wobei diese Substituenten gegebenenfalls mit dem Stickstoffatom einen 5- bis 7-gliedrigen Ring bilden, der als weiteres Heteroatom ein Sauerstoff- oder Schwefelatom oder eine N-Alkyl-Gruppierung mit 1 bis 4 C-Atomen enthalten kann,

$R^2$ für Wasserstoff, Cyano, Phenyl oder Benzyl, oder für geradkettiges oder verzweigtes Alkyl mit bis zu 4 Kohlenstoffatomen steht, das gegebenenfalls substituiert ist durch Acetoxy, Cyano, Hydroxy, ein oder mehrere Fluor, Chlor oder Brom,

$R^3$ für Wasserstoff, für geradkettiges oder verzweigtes, gegebenenfalls durch ein Sauerstoffatom unterbrochenes Alkyl mit bis zu 6 Kohlenstoffatomen, für Phenyl oder Benzyl steht,

$R^4$ für Wasserstoff, für geradkettiges oder verzweigtes Alkyl mit bis zu 6 C-Atomen,
für Phenyl, Benzyl oder
für einen Rest der Formel $CO-R^6$, $CO_2R^{6'}$, $CO-NR^6R^7$,
$SO_2R^{6'}$, $-CH_2-\underset{\displaystyle OH}{\underset{|}{CH}}-CH_2-OR^8$ steht,

wobei

$R^6$ und $R^7$ gleich oder verschieden sind und jeweils für Wasserstoff oder einen Kohlenwasserstoffrest von 1 bis 6 Kohlenstoffatomen, Phenyl oder Benzyl stehen,

$R^{6'}$ die Bedeutung von $R^6$ hat, jedoch nicht Wasserstoff bedeutet,

$R^8$ die für $R^5$ angegebene Bedeutung hat und mit diesem gleich oder verschieden sein kann

$R^5$ für Phenyl, Naphthyl oder für Furyl, Thienyl, Pyrryl, Pyrazolyl, Imidazolyl, Oxazolyl, Isoxazolyl, Thiazolyl, Thiadiazolyl, Pyridyl, Pyridazinyl, Pyrazinyl, Indolyl, Benzimidazolyl, Benzoxazolyl, Benzoxadiazolyl, Benzthiadiazolyl, Chinolyl, Isochinolyl, Chinazolyl, Chinoxazolyl oder Carbazolyl steht, wobei die

genannten Ringsysteme gegebenenfalls 1 bis 2 gleiche oder verschiedene Substituenten aus der Gruppe Phenyl, Alkyl (1 bis 8 C-Atome), Alkenyl (2 bis 8 C-Atome), Cycloalkyl (5 bis 7 C-Atome), Alkoxy (1 bis 4 C-Atome), Alkenyloxy (2 bis 4 C-Atome), Alkylen (2 bis 4 C-Atome), Morpholinyl, Dioxyalkylen (1 bis 3 C-Atome), Halogen, Polyfluroalkoxy (1 bis 4 C-Atome), Nitro, Cyano, Azido, $SO_m-R^6$, $-R^6R^7N-SO_m-$ (m = 0 oder 2), $NR^6R^7$, $NR^6COR^7$ und Acyl der Formel $CO-R^6$ enthalten können, wobei der Alkylsubstituent gegebenenfalls durch ein oder zwei Sauerstoffatome, Schwefelatome oder Gruppierungen der Formel $-NR^6-$, $-CONR^7-$ oder $-\underset{H}{N}CO-$ unterbrochen sein kann, und/oder durch Halogen substituiert sein kann, und wobei $R^6$ und $R^7$ jeweils die obengenannte Bedeutung besitzen,

A für eine geradkettige, verzweigte oder cyclische Alkylengruppe mit bis zu 20 Kohlenstoffatomen steht, die gegebenenfalls durch eine Phenylgruppe, welche ihrerseits durch Halogen, Cyano, Dialkylamino, Alkyl, Alkoxy mit je 1 bis 4 C-Atomen, Trifluormethyl oder Nitro substituiert sein kann, substituiert oder unterbrochen ist, und/oder die gegebenenfalls durch ein oder zwei Sauerstoffatome, Schwefelatome und Gruppierungen der Formel $-NR^6-$ oder $-CO-NR^7-$ unterbrochen ist, wobei $R^6$, $R^7$ die oben angegebene Bedeutung haben, und

X für die Gruppe $-COR^9$ steht, worin $R^9$ gegebenenfalls durch Halogen, Cyano, Alkoxy oder Dialkylamino mit jeweils 1 bis 4 C-Atomen substituiertes Alkyl mit 1 bis 7 C-Atomen, Phenyl, Naphthyl, Phenylalkyl oder Anilino oder eine Amino-, Monoalkylamino- oder Dialkylaminogruppe mit je 1 bis 4 C-Atomen bedeutet, wobei gegebenenfalls die Alkylgruppen mit dem Stickstoffatom einen 5- bis 7-gliedrigen Ring bilden, der als weiteres Heteroatom ein Sauerstoff- oder Schwefelatom enthalten kann,

oder für die Gruppe $-COOR^{10}$ steht, wobei $R^{10}$ die gleiche Bedeutung hat wie $R^1$ und mit diesem gleich oder verschieden sein kann,

sowie deren physiologisch unbedenklichen Säureadditionsprodukte.

Physiologisch unbedenkliche Säureadditionsprodukte können Salze der erfindungsgemäßen Verbindungen mit anorganischen oder organischen Säuren sein. Als Beispiele seien genannt: Salze mit Säuren wie Halogenwasserstoffsäuren, Schwefelsäure, Phosphorsäure, Sulfonsäuren, Essigsäure, Maleinsäure, Fumarsäure, Zitronensäure, Weinsäure, Milchsäure oder Benzoesäure.

Von ganz besonderem Interesse sind Verbindungen der allgemeinen Formel (I), in welcher

$R^{6'}$ die Bedeutung von $R^6$ hat, jedoch nicht Wasserstoff bedeutet,

$R^8$ die für $R^5$ angegebene Bedeutung hat und mit diesem gleich oder verschieden sein kann,

$R^5$ für Phenyl, Naphthyl oder Indolyl steht, wobei der Phenylrest gegebenenfalls durch 1 oder 2 gleiche oder verschiedene Substituenten aus der Gruppe Fluor, Chlor, Nitro, Cyano, Cyclopentyl, Cyclohexyl, Alkyl mit 1 bis 2 C-Atomen, Alkoxy mit 1 bis 2 C-Atomen, Allyl, Allyloxy, $NR^6R^7$, $NR^6COR^7$ oder Acetyl substituiert ist,

A für eine geradkettige, verzweigte oder cyclische Alkylengruppe mit bis zu 12 Kohlenstoffatomen steht, die gegebenenfalls durch eine Phenylgruppe substituiert oder unterbrochen ist und/oder die gegebenenfalls durch ein Sauerstoffatom oder eine Gruppierung der Formel $-NR^6-$ oder $-CONR^7-$ unterbrochen ist, wobei $R^6$, $R^7$ die angegebene Bedeutung haben,

und

X für die Gruppe $-COOR^{10}$ steht, wobei $R^{10}$ die gleiche Bedeutung hat wie $R^1$ und mit diesem gleich oder verschieden sein kann,

sowie deren physiologisch unbedenklichen Säureadditionsprodukte.

R für Phenyl, Thienyl, Furyl, Pyridyl oder Benzoxadiazolyl steht, wobei der Phenylrest 1 oder 2 gleiche oder verschiedene Substituenten aus der Gruppe Nitro, Trifluormethyl, Fluor, Chlor, Cyano, Alkoxy mit bis zu 4 C-Atomen oder Dioxymethylen enthält,

$R^1$ einen geradkettigen, verzweigten oder cyclischen Alkylrest mit bis zu 12 C-Atomen steht, der gegebenenfalls durch ein Sauerstoffatom in der Kette unterbrochen ist und/oder der gegebenenfalls substituiert ist durch Fluor, Chlor, Hydroxy, Pyridyl, Phenyl oder Amino,

$R^2$ für Phenyl, Benzyl, Methyl, Ethyl, Acetoxymethyl, Trifluormethyl, Chlormethyl oder Brommethyl steht,

$R^3$ für Wasserstoff oder Alkyl mit 1 bis 2 Kohlenstoffatomen steht,

$R^4$ für Wasserstoff,
für geradkettiges oder verzweigtes Alkyl mit bis zu 4 C-Atomen, für Benzyl oder
für einen Rest der Formel $-CO-R^6$, $-CO_2R^{6'}$, $CO-NR^6R^7$, $SO_2R^{6'}$, $-CH_2-\underset{\displaystyle OH}{\underset{|}{C}}H-CH_2-OR^8$ steht,

wobei

$R^6$ und $R^7$ gleich oder verschieden sind und jeweils für Wasserstoff, Alkyl mit bis zu 4 C-Atomen, Phenyl oder Benzyl steht,

Für das erfindungsgemäße Verfahren kommen alle inerten organischen Lösungsmittel in Frage. Hierzu gehören vorzugsweise niedere Alkohole wie Ethanol, Methanol, Propanol oder Isopropanol, Ether wie Dioxan, Diethylether, Tetrahydrofuran oder Glykolmonomethylether sowie chlorierte Kohlenwasserstoffe wie Dichlormethan, Chloroform und 1,2-Dichlorethan, sowie Gemische dieser Lösungsmittel.

Die Reaktionstemperaturen können in einem größeren Bereich variiert werden. Im allgemeinen arbeitet man zwischen 10 und 150°C, vorzugsweise zwischen 20 und 130°C, insbesondere bei der Siedetemperatur des jeweiligen Lösungsmittels.

Die Umsetzung kann bei Normaldruck, aber auch bei erniedrigtem oder erhöhtem Druck durchgeführt werden. Im allgemeinen arbeitet man unter Normaldruck.

Für die Synthese der Verbindungen der Formel (I) mit $R^4$ = Wasserstoff, werden die entsprechenden Amine der Formel (II) in molaren bis doppelt molaren Mengen eingesetzt.

Für die weitere Reaktion zu Verbindungen der Formel (I), in welcher $R^4$ für den Rest $-CH_2-\underset{\displaystyle OH}{\underset{|}{CH}}-CH-OR^8$ steht,

werden die entsprechenden Epoxide der Formel (III) in molaren bis dreifach molaren Mengen eingesetzt.

Für die Acylierung bzw. Sulfonylierung werden die Reaktanden in molaren Mengen eingesetzt.

Das vorstehende Herstellungsverfahren ist lediglich zur Verdeutlichung angegeben, und die Herstellung der Verbindung der Formel (I) ist nicht auf dieses Verfahren beschränkt, sondern jede Modifikation dieses Verfahrens ist in gleicher Weise für die Herstellung der erfindungsgemäßen Verbindung anwendbar.

Je nach Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Die als Ausgangsstoffe verwendeten Dihydropyridine der allgemeinen Formel (II) sind teilweise bekannt (EP-OS 89 167).

Die Erfindung betrifft ebenfalls neue als Ausgangsstoffe verwendete Dihydropyridine der allgemeinen Formel (IIa)

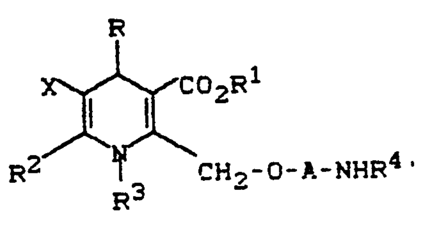

(IIa),

in welcher

R für Aryl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei der Arylrest sowie die Heterocyclen gegebenenfalls 1 bis 2 gleiche oder verschiedene Substituenten aus der Gruppe Phenyl, Alkyl, Alkoxy, Alkylen, Dioxyalkylen, Halogen, Trifluormethyl, Polyfluoralkoxy, Nitro, Cyano, Azido oder $SO_m$-Alkyl (m = 0 bis 3) enthalten,

$R^1$ einen geradkettigen, verzweigten oder cyclischen, gesättigten oder ungesättigten Kohlenwasserstoffrest bedeutet, der gegebenenfalls durch ein Sauerstoff- oder ein Schwefelatom in der Kette unterbrochen ist, und/oder der gegebenenfalls substituiert ist durch Halogen, Cyan, Hydroxy, Acyloxy, Pyridyl oder durch ein Phenyl-, Phenoxy-, Phenylthio- oder Phenylsulfonylgruppe, wobei die Arylreste ihrerseits wieder durch Halogen, Cyano, Dialkylamino, Alkoxy, Alkyl, Trifluormethyl oder Nitro substituiert sein können,

oder der gegebenenfalls durch eine Aminogruppe substituiert ist, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem

Stickstoffatom einen 5- bis 7-gliedrigen Ring bilden, der als weiteres Heteroatom ein Sauerstoff- oder Schwefelatom oder eine N-Alkyl-Gruppierung enthalten kann,

$R^2$ für Wasserstoff, Cyano, Aryl, Aralkyl oder für geradkettiges oder verzweigtes Alkyl steht, das gegebenenfalls substituiert ist durch Cyano, Hydroxy, Acetoxy oder Halogen,

$R^3$ für Wasserstoff oder geradkettiges oder verzweigtes Alkyl steht, das gegebenenfalls durch ein oder zwei Sauerstoffatome in der Alkylkette unterbrochen ist, oder für Aryl oder Aralkyl steht,

$R^{4'}$ für Wasserstoff, Alkyl, Aryl oder Aralkyl steht,

A für eine geradkettige, verzweigte oder cyclische Alkylengruppe mit bis zu 20 Kohlenstoffatomen steht, die durch eine gegebenenfalls durch Halogen, Cyano, Dialkylamino, Alkyl, Alkoxy, Trifluormethyl oder Nitro substituierte Phenylgruppe substituiert oder unterbrochen sein kann, und/oder die gegebenenfalls durch ein oder mehrere Sauerstoffatome, Schwefelatome oder Gruppierungen der Formel $-NR^6-$ oder $-CO-NR^7-$ unterbrochen ist, wobei

$R^6$ und $R^7$ gleich oder verschieden sind und jeweils für Wasserstoff, einen niedrigen Kohlenwasserstoffrest, Aryl oder Aralkyl stehen, und

wobei

A jedoch nicht für die Gruppen $-(CH_2)_2-$, $-(CH_2)_3-$, $-CH_2CH(CH_3)-$ und $-CH_2C(CH_3)_2-$ stehen darf,

und

X für die Gruppe $-COR^9$ steht, worin $R^9$ gegebenenfalls substituiertes Alkyl, Aryl, Aralkyl oder Anilino oder eine Amino-, Monoalkylamino- oder Dialkylaminogruppe bedeutet, wobei gegebenenfalls die Alkylgruppen mit dem Stickstoffatom einen 5- bis 7-gliedrigen Ring bilden, der als weiteres Heteroatom ein Sauerstoff- oder Schwefelatom enthalten kann,

oder für die Gruppe $-COOR^{10}$ steht, worin $R^{10}$ die gleiche Bedeutung hat wie $R^1$ und mit diesem gleich oder verschieden sein kann,

sowie deren Säureadditionsprodukte.

Die Dihydropyridine der allgemeinen Formel (II) können hergestellt werden, indem man Verbindungen der Formel (IV)

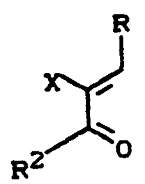

(IV),

in welcher

R, $R^2$ und X die oben angegebene Bedeutung haben, mit Enaminen der allgemeinen Formel (V),

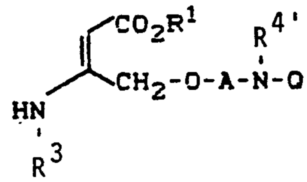

(V),

in welcher

$R^1$, $R^3$, $R^{4'}$ und A die oben angegebene Bedeutung haben und

Q für eine Aminoschutzgruppe, bevorzugt für tert.-Butyloxycarbonyl oder Benzyl steht, oder $R^{4'}$ und Q gemeinsam für Phthalimid stehen,

in inerten Lösungsmitteln umsetzt und anschließend in bekannter Weise die Aminoschutzgruppe abspaltet.

Als Lösungsmittel kommen alle inerten organischen Lösungsmittel in Frage. Hierzu gehören vorzugsweise niedere Alkohole wie Methanol, Ethanol, Propanol oder Isopropanol, Ether wie Dioxan, Tetrahydrofuran oder Diethylether, sowie chlorierte Kohlenwasserstoffe wie Di-, Tri- oder Tetrachlormethan, 1,2-Dichlorethan oder Trichlorethylen.

Die Reaktionstemperaturen können in einem größeren Bereich variiert werden. Im allgemeinen arbeitet man zwischen 10 und 150°C, vorzugsweise zwischen 20 und 130°C, insbesondere bei der Siedetemperatur des jeweiligen Lösungsmittels.

Die Umsetzung kann bei Normaldruck aber auch bei erhöhtem oder erniedrigtem Druck durchgeführt werden. Im allgemeinen arbeitet man bei Normaldruck.

Die Abspaltung der Aminoschutzgruppe erfolgt in an sich bekannter Weise, beispielsweise durch Hydrierung mit Hilfe von Palladium/Tierkohle unter sauren Bedingungen, wenn Q für Benzyl steht, oder wenn Q für den Phthalimidrest steht, erfolgt die Abspaltung der Schutzgruppe üblicherweise mit Hydrazinhydrat in organischen Lösungsmitteln wie Ethern, z.B. Tetrahydrofuran oder Dioxan, oder Alkolen z.B. Methanol, Ethanol oder Isopropanol.

Die Ylidenverbindungen der allgemeinen Formel (IV) sind bekannt oder können nach bekannten Methoden hergestellt werden [G.Jones "The Knoevenagel Condensation", Organic Reactions XV, 204 (1967)]. Die Enamine der allgemeinen Formel (V) sind bekannt oder können nach bekannten Methoden hergestellt werden [EP-OS 89 167].

Als Beispiele für die erfindungsgemäß als Zwischenprodukte verwendeten Dihydropyridine der allgemeinen Formel (II) seien genannt:

2-[(2-Aminoethoxy)methyl]-4-(2-chlorphenyl)-3-ethoxycarbonyl-5-methoxycarbonyl-6-methyl-1,4-dihydropyridin
2-[(2-Aminoethoxy)methyl]-3-ethoxycarbonyl-5-butoxycarbonyl-6-methyl-4-(3-nitrophenyl)-1,4-dihydropyridin

2-[(2-Aminoethoxy)methyl]-4-(2-chlorphenyl)-4-methoxycarbonyl-5-isopropoxycarbonyl-6-methyl-1,4-dihydropyridin

2-[(2-Aminoethoxy)methyl]-4-(2-chlorphenyl)-3-ethoxy-carbonyl-5-isopropoxycarbonyl-6-methyl-1,4-dihydropyridin

2-[(2-Aminoethoxy)methyl]-4-benzoxadiazolyl-3-ethoxycarbonyl-5-isopropoxycarbonyl-6-methyl-1,4-dihydropyridin

2-(2S-Aminopropoxy)-methyl]-4-(2-chlorphenyl)-3-ethoxycarbonyl-5-methoxycarbonyl-6-methyl-1,4-dihydropyridin

2-[(2S-Aminobutoxy)methyl]-4-(2-chlorphenyl)-3-ethoxycarbonyl-5-methoxycarbonyl-6-methyl-1,4-dihydropyridin

2-[(2S-Amino-2S-phenyl-ethoxy)methyl]-3-ethoxycarbonyl-5-methoxycarbonyl-6-methyl-4-(2-trifluormethylphenyl)-1,4-dihydropyridin

2-[(2R-Amino-2R-phenyl-ethoxy)methyl]-4-(2-chlorphenyl-3-isopropoxycarbonyl-5-methoxycarbonyl-6-methyl-1,4-dihydropyridin.

Die als Ausgangsstoffe verwendeten Epoxide der allgemeinen Formel (III) bzw. der Formel (IIIa) sind literaturbekannt oder können nach literaturbekannten Methoden hergestellt werden (vgl. A.F. Growther and L.H. Gnuth, J. Med. Chem. 11, 1009 (1968)).

Als Beispiele seien genannt:

1-Phenoxy-2,3-epoxy-propan

1-(4-Methoxyphenoxy)-2,3-epoxy-propan,

1-(3-Methoxyphenoxy)-2,3-epoxy-propan,

1-(3-Methoxyphenoxy)-2,3-epoxy-propan,

1-(2-Allylphenoxy)-2,4-epoxy-propan,

1-(2-Allyloxyphenoxy)-2,3-epoxy-propan,

1-(4-Carbazolyloxy)-2,3-epoxy-propan,
1-(4-Methoxyethylphenoxy)-2,3-epoxy-propan,
1-(4-Indolyloxy)-2,3-epoxy-propan,
1-(1-Naphthyloxy)-2,3-epoxy-propan
1-(4-Allyl-3-methoxyphenoxy)-2,3-epoxy-propan,
1-(2-Cyanophenoxy)-2,3-epoxy-propan,
1-(5-Allyl-2-ethoxyphenoxy)-2,3-epoxy-propan,
1-(4-Aminocarbonylmethyl-phenoxy)-2,3-epoxy-propan,
1-(4-Methylsulfonyl-N-methylamido-phenoxy)-2,3-epoxy-propan,
1-(4-Methyl-carbaminoylethyl-phenoxy)-2,3-epoxy-propan,
1-(2-Acetyl-4-propionamido-phenoxy)-2,3-epoxy-propan,
1-(4-Acetamidophenoxy)-2,3-epoxy-propan.

Die neuen erfindungsgemäßen Verbindungen der Formel (I) haben ein breites und vielseitiges pharmakologisches Wirkungsspektrum. Sie besitzen calciumantagonistische und ß-Adrenorezeptoren-blockierende Eigenschaften. Überraschenderweise hemmen sie die KCl- bzw. Noradrenalin-induzierten Kontraktionen von isolierten Kaninchenaorten (Versuchsanordnung: R. Towart, J. Cardiovascular Pharmacology 4, 895-902 (1982).

Im einzelnen konnten im Tierexperiment folgende Hauptwirkungen nachgewiesen werden:

1. Die Verbindungen bewirken bei parenteraler, oraler und perlingualer Gabe eine deutliche Erweiterung der Coronargefäße. Diese Wirkung auf die Coronargefäße wird durch einen gleichzeitigen herzentlastenden

Effekt verstärkt. Durch die zusätzliche antagonisierende Wirkung auf die ß-adrenergen Rezeptoren hemmen die Verbindungen eine pathologisch gesteigerte Kontraktilität des Herzens. Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare antiarrhythmische Wirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich in mehr oder weniger isolierten umschriebenen Gefäßgebieten (wie z.B. dem Zentralnervensystem oder in der Niere) manifestieren. Die Verbindungen eignen sich daher besonders als Cerebraltherapeutika und als Mittel zur Behandlung von Nierenfunktionsstörungen.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, die an der glatten Muskulatur des Magens, Darmtraktes, des Urogenitaltraktes und des Respirationssystems deutlich werden.

6. Die Verbindungen reduzieren die Herzfrequenz.

Die erfindungsgemäßen Verbindungen eignen sich aufgrund dieser Eigenschaften besonders zur Prophylaxe und Therapie der akuten und chronischen ischämischen Herzkrankheit im weitesten Sinne, zur Therapie des Hochdrucks, zur Behandlung von cerebralen, renalen und systemischen Durchblutungsstörungen sowie zur Behandlung von Herzrhythmusstörungen, Schwangerschaftshochdruck, Phäochromozytom, hypertrophischen Kardiomyophatien, hyperkinetischem Herzsyndrom und zur Kardioprotektion beim akuten Herzinfarkt und bei Kardioplegie als auch zur Prävention des Sekundärinfarktes.

Außerdem können die Verbindungen als Zusatzmedikation zur symptomatischen Behandlung von Hyperthyreose, Parkinsonismus, Angstzuständen, Migräne, psychosomatischen Störungen und Glaukom angewandt werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen

und/oder Elixieren, die für orale Anwendungen gedacht sind, können die Wirkstoffe außer mit den obengenannten Hilfsstoffen mit verschiedenen Geschmacksaufbessern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 5 mg/kg, vorzugweise etwa 0,05 bis 2 mg/kg Körpergewicht pro Tag zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele:

Beispiel 1

2- (2-[2-Hydroxy-3-(1-naphthyloxy)-1-propyl]-aminoethoxy)-methyl -4-(2-chlorphenyl)-3-ethoxycarbonyl-5-methoxycarbonyl-6-methyl-1,4-dihydropyridin.

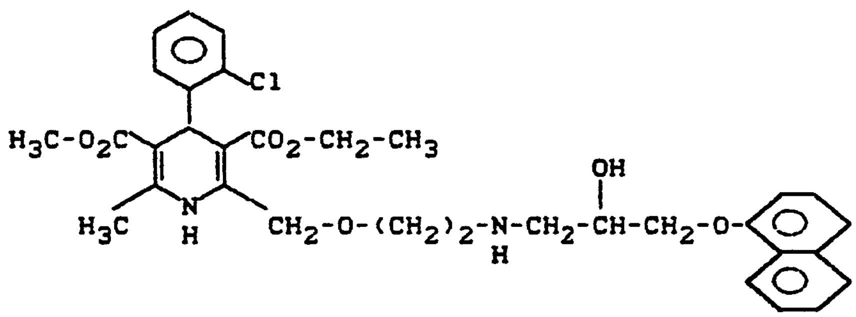

Eine Lösung von 8,2 g (20,0 mmol) 2-[(2-Aminoethoxy)-methyl]-4-(2-chlorphenyl)-3-ethoxycarbonyl-5-methoxycarbonyl-6-methyl-1,4-dihydropyridin in 80 ml Isopropanol wurde mit 2,0 g (100 mmol) 1-(1-Naphthyloxy)-2,3-epoxypropan versetzt und 24 Stunden unter Rückfluß gerührt.

Anschließend wurde im Vakuum eingeengt und der gebildete Rückstand durch Säulenchromatographie an Kieselgel (40-63 µm) mit dem Laufmittelgemisch Chloroform/Methanol/$NH_3$ = 20:1:0,05 (System 1) gereinigt.

Das nach Einengen im Vakuum erhaltene amorphe Pulver wurde im Vakuum bei 40°C getrocknet.
Ausbeute: 4,46 g (73,1 %)

<u>Analyse:</u>

| | C | H | Cl | N |
|---|---|---|---|---|
| Ber. | 65,07 | 6,12 | 5,82 | 4,59 |
| Gef. | 65,12 | 6,15 | 5,82 | 4,61 |

Die in der folgenden Tabelle aufgeführten Beispiele werden analog Bsp. 1 hergestellt

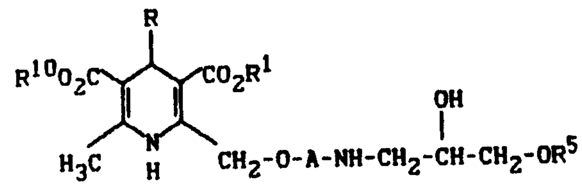

| Bsp. | R | $R^1$ | $R^{10}$ | A | $R^5$ | Analyse | | | | | Ausbeute % d.Theorie | Rf |
|---|---|---|---|---|---|---|---|---|---|---|---|---|
| 2 | 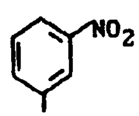 | $-C_2H_5$ | $-CH_3$ | $-CH_2CH_2-$ | 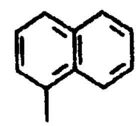 | ber. | C 63,96 | H 6,02 | N 6,78 | | 68,5 | 0,44 |
| | | | | | | gef. | 63,85 | 6,01 | 6,75 | | | |
| 3 | 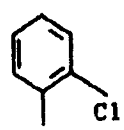 | $-CH_3$ | $-CH(CH_3)_2$ | $-CH_2CH_2-$ | 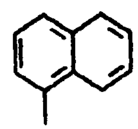 | ber. | C 65,53 | H 6,31 | Cl 5,69 | N 4,49 | 56,0 | 0,44 |
| | | | | | | gef. | 64,47 | 6,28 | 5,72 | 4,51 | | |
| 4 | 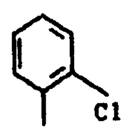 | $-C_2H_5$ | $-CH(CH_3)_2$ | $-CH_2-CH_2-$ | 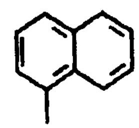 | ber. | C 65,97 | H 6,48 | Cl 5,56 | N 4,42 | 81,4 | 0,40 |
| | | | | | | gef. | 65,94 | 6,50 | 5,58 | 4,45 | | |
| 5 | 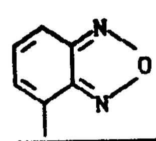 | $-C_2H_5$ | $CH(CH_3)_2$ | $-CH_2-CH_2-$ | 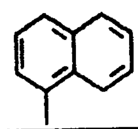 | ber. | C 65,20 | H 6,25 | N 8,69 | | 72,3 | 0,43 |
| | | | | | | gef. | 65,18 | 6,29 | 8,73 | | | |

| Bsp. | R | $R^1$ | $R^{10}$ | A | $R^5$ | Analyse | C | H | Cl | N | Ausbeute % d.Theorie | Rf |
|---|---|---|---|---|---|---|---|---|---|---|---|---|
| 6 | 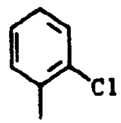 | $-C_2H_5$ | $-CH_3$ | $-CH_2-CH_2-$ | 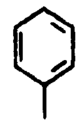 | ber. | 62,30 | 6,31 | 6,34 | 5,01 | 61,7 | 0,37 |
| | | | | | | gef. | 62,45 | 6,40 | 6,28 | 5,03 | | |
| 7 | 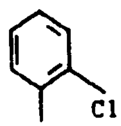 | $-C_2H_5$ | $-CH_3$ | $-CH_2-CH_2-$ | 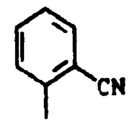 | ber. | 61,69 | 5,87 | 6,07 | 7,19 | 56,2 | 0,37 |
| | | | | | | gef. | 61,73 | 5,90 | 6,05 | 7,22 | | |
| 8 | 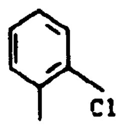 | $-C_2H_5$ | $-CH_3$ | $-CH_2-CH_2-$ | 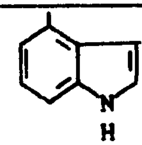 | ber. | 65,25 | 6,07 | 5,93 | 7,03 | 64,8 | 0,30 |
| | | | | | | gef. | 64,98 | 6,09 | 5,84 | 7,07 | | |
| 9 | 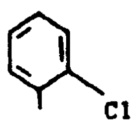 | $-C_2H_5$ | $-CH_3$ | $-CH_2-CH_2$ | 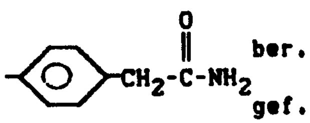 | ber. | 60,23 | 6,52 | 5,74 | 6,79 | 49,1 | 0,12 |
| | | | | | | gef. | 60,33 | 6,74 | 5,90 | 6,83 | | |
| 10 | 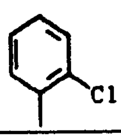 | $-C_2H_5$ | $-CH_3$ | $-CH_2-\overset{CH_3}{CH}-$ | 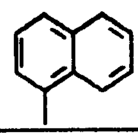 | ber. | 65,53 | 6,31 | 5,69 | 4,49 | 80,3 | 0,43 |
| | | | | | | gef. | 64,97 | 6,34 | 5,75 | 4,38 | | |

| Bsp. | R | $R^1$ | $R^{10}$ | A | $R^5$ | Analyse | C | H | Cl | N | Ausbeute % d.Theorie | Rf |
|---|---|---|---|---|---|---|---|---|---|---|---|---|
| 11 | 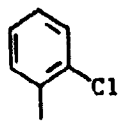 | $-C_2H_5$ | $-CH_3$ | 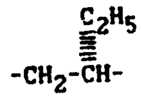 | 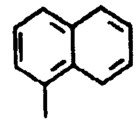 | ber. | 65,98 | 6,49 | 5,56 | 4,39 | 67,6 | 0,51 |
| | | | | | | gef. | 65,83 | 6,39 | 5,60 | 4,32 | | |
| 12 | 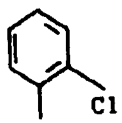 | $-C_2H_5$ | $-CH_3$ | 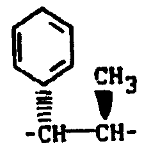 | 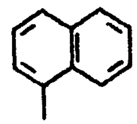 | ber. | 68,71 | 6,19 | 5,07 | 4,01 | 52,8 | 0,56 |
| | | | | | | gef. | 69,01 | 6,23 | 5,15 | 4,08 | | |
| 13 | 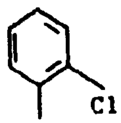 | $-C_2H_5$ | $-CH_3$ | 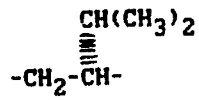 | 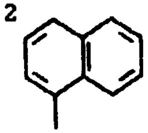 | ber. | 66,39 | 6,66 | 5,44 | 4,30 | 48,9 | 0,53 |
| | | | | | | gef. | 66,48 | 6,59 | 5,60 | 4,19 | | |
| 14 | 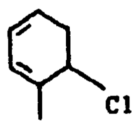 | $-C_2H_5$ | $-CH_3$ | $-(CH_2)_2-O-(CH_2)_2-$ | 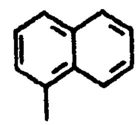 | ber. | 64,36 | 6,33 | 5,43 | 4,29 | 70,5 | 0,37 |
| | | | | | | gef. | 64,72 | 6,41 | 5,38 | 4,35 | | |
| 15 | 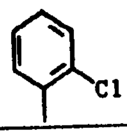 | $-C_2H_5$ | $-CH_3$ | 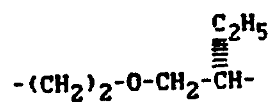 | 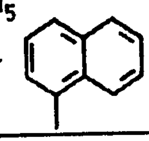 | ber. | 65,24 | 6,66 | 5,20 | 4,11 | 62,7 | 0,43 |
| | | | | | | gef. | 65,72 | 6,80 | 5,13 | 4,22 | | |

| Bsp. | R | $R^1$ | $R^{10}$ | A | $R^5$ | Analyse | | | | | Ausbeute % d.Theorie | Rf |
|---|---|---|---|---|---|---|---|---|---|---|---|---|
| | | | | | | | C | H | Cl | N | | |
| 16 | 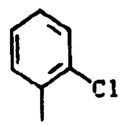 | $-C_2H_5$ | $-CH_3$ | $-(CH_2)_5-$ | 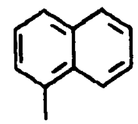 | ber. | 66,39 | 6,65 | 5,44 | 4,30 | 61,0 | 0,35 |
| | | | | | | gef. | 66,50 | 6,72 | 5,38 | 4,21 | | |
| | | | | | | | C | H | Cl | N | | |
| 17 | 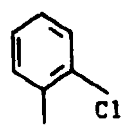 | $-C_2H_5$ | $-CH_3$ | $-(CH_2)_6-$ | 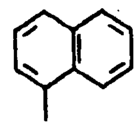 | ber. | 66,80 | 6,81 | 5,32 | 4,21 | 58,1 | 0,38 |
| | | | | | | gef. | 66,54 | 6,93 | 5,40 | 4,32 | | |
| | | | | | | | C | H | Cl | N | | |
| 18 | 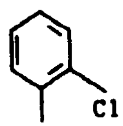 | $-C_2H_5$ | $-CH_3$ | $-(CH_2)_{11}-$ | 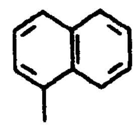 | ber. | 68,59 | 7,53 | 4,82 | 3,80 | 66,3 | 0,4 |
| | | | | | | gef. | 69,01 | 7,49 | 4,76 | 3,90 | | |
| | | | | | | | C | H | F | N | | |
| 19 | 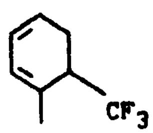 | $-C_2H_5$ | $-C_2H_5$ | 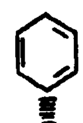 $-CH_2-CH-$ | 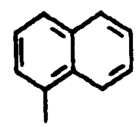 | ber. | 67,10 | 6,04 | 7,76 | 3,81 | 42,8 | 0,69 |
| | | | | | | gef. | 67,23 | 6,09 | 7,82 | 3,94 | | |
| | | | | | | | C | H | F | N | | |
| 20 | 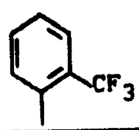 | $-C_2H_5$ | $-C_2H_5-(CH_2)_2-N(CH_3)-(CH_2)_3-$ | | 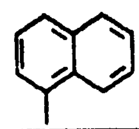 | ber. | 64,35 | 6,64 | 7,83 | 5,77 | 73,6 | 0,32 |
| | | | | | | gef. | 64,95 | 6,58 | 7,92 | 5,69 | | |

| Bsp. | R | $R^1$ | $R^{10}$ | A | $R^5$ | Analyse | C | H | N | F | S | Cl | Ausbeute % d.Theorie | Rf |
|---|---|---|---|---|---|---|---|---|---|---|---|---|---|---|
| 21 | 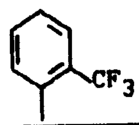 | $-C_2H_5$ | $-C_2H_5$ | $-(CH_2)_2-S-(CH_2)_2-$ | 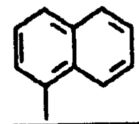 | ber.<br>gef. | 61,99<br>61,83 | 6,04<br>6,10 | 3,90<br>3,93 | 7,95<br>7,88 | | | 39,8 | 0,48 |
| 22 | 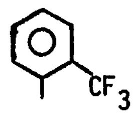 | $CH_3-CH_2-$ | $CH_3-CH_2-$ | $-(CH_2)_2-S-(CH_2)_2-$ | 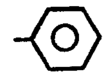 | ber.<br>gef. | 59,4<br>59,2 | 6,2<br>6,1 | 4,2<br>4,1 | 8,5<br>8,4 | 4,8<br>4,6 | | 68,6 | 0,358 |
| 23 | 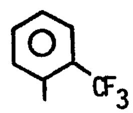 | $CH_3-CH_2-$ | $CH_3-CH_2-$ | $-(CH_2)_2-N(CH_3)-(CH_2)_3-$ | 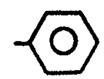 | ber.<br>gef. | 62,0<br>61,7 | 6,8<br>6,7 | 6,2<br>6,1 | 8,4<br>8,4 | -<br>- | | 81,2 | 0,242 |
| 24 | 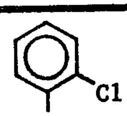 | $-C_2H_5$ | $-CH_3$ | $-(CH_2)_{11}-$ | 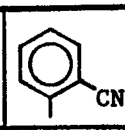 | ber.<br>gef. | 65,98<br>65,5 | 7,32<br>7,22 | 5,91<br>5,75 | | | 4,99<br>5,23 | 67,3 | 0,43 |
| 25 | 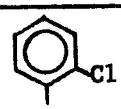 | $-C_2H_5$ | $-CH_3$ | $-(CH_2)_2-O-(CH_2)_2$ | 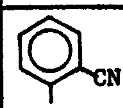 | ber.<br>gef. | 61,25<br>60,9 | 6,05<br>5,98 | 6,69<br>6,59 | | | 5,64<br>5,8 | 73,4 | 0,33 |
| 26 | 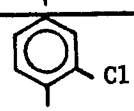 | $-C_2H_5$ | $-CH_3$ | $-(CH_2)_6-$ | 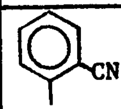 | ber.<br>gef. | 63,86<br>63,5 | 6,56<br>6,49 | 6,56<br>6,52 | | | 5,54<br>5,7 | 82,3 | 0,24 |

| Bsp. | R | $R^1$ | $R^{10}$ | A | $R^5$ | Analyse | C | H | N | Cl | Ausbeute % d.Th. | $R_f$ |
|---|---|---|---|---|---|---|---|---|---|---|---|---|
| 27 | 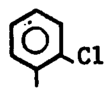 | $-C_2H_5$ | $-CH_3$ | $-CH_2-CH(C_2H_5)-$ | 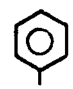 | Ber. | 63,42 | 6,70 | 6,04 | 4,77 | 68,1 | 0,50 |
| | | | | | | Gef. | 62,9 | 6,1 | 5,9 | 4,8 | | |
| 28 | 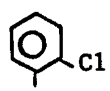 | $-CH_3$ | $-CH(CH_3)_2$ | $-CH_2-CH_2-$ | 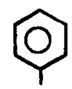 | Ber. | 62,87 | 6,51 | 6,19 | 4,89 | 72,3 | 0,47 |
| | | | | | | Gef. | 62,5 | 6,4 | 6,2 | 4,8 | | |
| 29 | 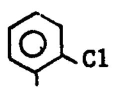 | $-CH_3$ | $-CH(CH_3)_2$ | $-CH_2-CH_2-$ | 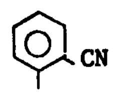 | Ber. | 62,25 | 6,07 | 5,93 | 7,03 | 59,8 | 0,47 |
| | | | | | | Gef. | 61,9 | 5,9 | 5,8 | 6,9 | | |
| 30 | 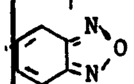 | $-C_2H_5$ | $-CH(CH_3)_2$ | $-CH_2-CH_2-$ | 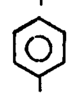 | Ber. | 62,61 | 6,44 | 9,42 | | 65,4 | 0,44 |
| | | | | | | Gef. | 62,1 | 6,2 | 9,5 | | | |
| 31 | 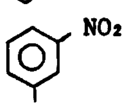 | $-C_2H_5$ | $-CH_3$ | $-CH_2-CH_2-$ | 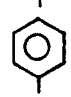 | Ber. | 61,15 | 6,19 | 7,38 | | 75,1 | 0,45 |
| | | | | | | Gef. | 60,8 | 6,0 | 7,5 | | | |
| 32 | 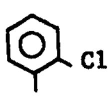 | $-C_2H_5$ | $-CH_3$ | $-CH_2-CH_2-$ | 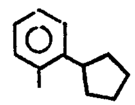 | Ber. | 65,11 | 6,91 | 5,65 | 4,47 | 61,9 | 0,51 |
| | | | | | | Gef. | 65,3 | 7,0 | 5,5 | 4,5 | | |

| Bsp. | R | $R^1$ | $R^{10}$ | A | $R^5$ | Analyse | C | H | N | Cl | Ausbeute % d.Th. | $R_f$ |
|---|---|---|---|---|---|---|---|---|---|---|---|---|
| 33 | 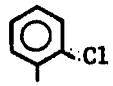 | $-C_2H_5$ | $-CH_3$ | $-(CH_2)_{11}-$ | 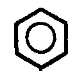 | Ber. | 66,62 | 7,74 | 4,09 | 5,18 | 67,4 | 0,432 |
| | | | | | | Gef. | 66,0 | 7,1 | 4,0 | 5,3 | | |
| 34 | 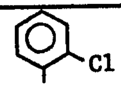 | $-C_2H_5$ | $-CH_3$ | $-CH_2-\overset{CH_3}{CH}-$ | 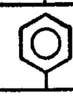 | Ber. | 62,88 | 6,46 | 4,89 | 6,19 | 93,79 | 0,436 |
| | | | | | | Gef. | 62,25 | 6,1 | 4,7 | 6,2 | | |
| 35 | 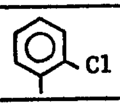 | $-C_2H_5$ | $-CH_3$ | $-(CH_2)_2-O-(CH_2)_2-$ | 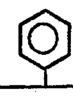 | Ber. | 61,74 | 6,47 | 4,64 | 5,88 | 82,14 | 0,348 |
| | | | | | | Gef. | 61,5 | 6,31 | 4,47 | 5,9 | | |
| 36 | 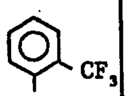 | $-C_2H_5$ | $-C_2H_5$ | 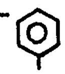 $-CH_2-CH$ | 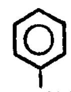 | Ber. | 65,16 | 6,01 | 4,105 | F 2,78 | 61,3 | 0,637 |
| | | | | | | Gef. | 64,87 | 5,79 | 4,03 | 2,6 | | |
| 37 | 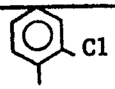 | $-C_2H_5$ | $-CH_3$ | $-(CH_2)_5$ | 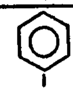 | Ber. | 63,95 | 6,828 | 4,66 | 5,9 | 69,5 | 0,315 |
| | | | | | | Gef. | 63,49 | 6,62 | 4,37 | 6,12 | | |
| 38 | 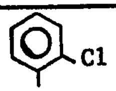 | $-C_2H_5$ | $-CH_3$ | $-(CH_2)_6$ | 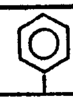 | Ber. | 64,44 | 6,99 | 4,55 | 5,76 | 75,6 | 0,308 |
| | | | | | | Gef. | 64,12 | 6,73 | 4,32 | 5,85 | | |

Laufmittel für Beispiele 1-9, 11-13 und 27-32:

$CHCl_3$ 20 Gewichtsteile
$CH_3OH$ 1 Gewichtsteil
$NH_3$ 0,05 Gewichtsteile

Laufmittel für Beispiele 10, 14, 16-21, 24-26 und 33-38:

$CH_2Cl_2$ 100 Gewichtsteile
$CH_3CH$ 10 Gewichtsteile
$NH_3$ 1 Gewichtsteil

Laufmittel für Beispiele 22 und 23:

$CH_2Cl_2$ 100 Gewichtsteile
$H_3COH$ 10 Gewichtsteile

Beispiel 39 (Zwischenprodukt)

2-[2S-Aminobutoxy)methyl]-4-(2-Chlorphenyl)-3-ethoxycarbonyl-5-methoxycarbonyl-6-methyl-1,4-dihydropyridin

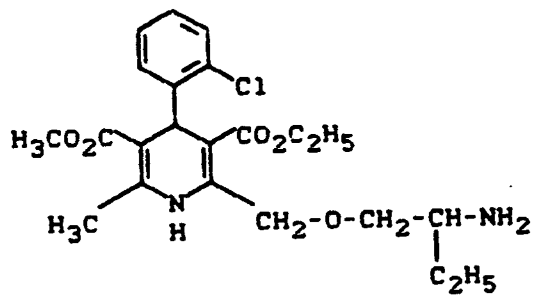

Eine Lösung von 56,7 g (100 mmol) 4-(2-Chlorphenyl)-3-ethoxycarbonyl-5-methoxycarbonyl-6-methyl-2-(2S-phthalimidobutoxy)methyl-1,4-dihydropyridin und 25 g Hydrazinhydrat in 1000 ml Ethanol wurde zwei Stunden unter Rückfluß gekocht.

Der entstandene Niederschlag wurde heiß filtriert und das Filtrat am Rotationsverdampfer auf ein Drittel des Volumens eingeengt. Nach Zugabe von 500 ml Methylenchlorid wurde mit 500 ml Wasser gewaschen. Die organische Phase wurde mit Natriumsulfat getrocknet und im Vakuum eingeengt. Das so erhaltene amorphe Produkt wurde bei 40°C im Ölpumpen-Vakuum getrocknet.

Ausbeute: 23,2 g (53,1 %)

| | C | H | Cl | N |
|---|---|---|---|---|
| ber. | 60,47 | 6,69 | 8,11 | 6,41 |
| gef. | 60,58 | 6,72 | 8,34 | 6,50 |

Patentansprüche:

1. Verbindungen der allgemeinen Formel I

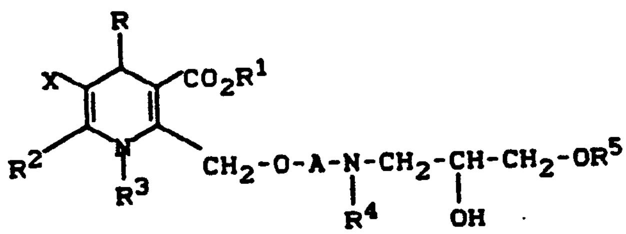

(I)

in welcher

R für Aryl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei der Arylrest sowie die Heterocyclen gegebenenfalls 1 bis 2 gleiche oder verschiedene Substituenten aus der Gruppe Phenyl, Alkyl, Alkoxy, Alkylen, Dioxyalkylen, Halogen, Trifluormethyl, Polyfluoralkoxy, Nitro, Cyano, Azido oder $SO_m$-Alkyl ($m = 0$ bis 3) enthalten,

$R^1$ einen geradkettigen, verzweigten oder cyclischen, gesättigten oder ungesättigten Kohlenwasserstoffrest bedeutet, der gegebenenfalls durch ein Sauerstoff- oder ein Schwefelatom in der Kette unterbrochen ist,

und/oder der gegebenenfalls substituiert ist durch Halogen, Cyan, Hydroxy, Acyloxy, Pyridyl oder durch eine Phenyl-, Phenoxy-, Phenylthio- oder Phenylsulfonylgruppe, wobei die Arylreste ihrerseits wieder durch Halogen, Cyano, Dialkylamino, Alkoxy, Alkyl, Trifluormethyl oder Nitro substituiert sein können,

oder der gegebenenfalls durch eine Aminogruppe substituiert ist, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem Stickstoffatom einen 5- bis 7-gliedrigen Ring bilden, der als weiteres Heteroatom ein Sauerstoff- oder Schwefelatom oder eine N-Alkyl-Gruppierung enthalten kann,

$R^2$ für Wasserstoff, Cyano, Aryl, Aralkyl oder für geradkettiges oder verzweigtes Alkyl steht, das gegebenenfalls substituiert ist durch Cyano, OH, Acetoxy oder Halogen,

$R^3$ für Wasserstoff oder geradkettiges oder verzweigtes Alkyl steht, das gegebenenfalls durch ein oder zwei Sauerstoffatome in der Alkylkette unterbrochen ist, oder für Aryl oder Aralkyl steht,

$R^4$ für Wasserstoff, für geradkettiges oder verzweigtes Alkyl, für Aryl, Aralkyl oder für einen Rest der Formel $CO-R^6$, $CO_2-R^{6'}$, $CO-NR^6R^7$, $SO_2R^6$, $-CH_2-\underset{\displaystyle OH}{\underset{|}{CH}}-CH_2-OR^8$ steht,

wobei

$R^6$ und $R^7$ gleich oder verschieden sind und jeweils für Wasserstoff, einen niedrigen Kohlenwasserstoffrest, Aryl oder Aralkyl stehen,

$R^{6'}$ die Bedeutung von $R^6$ hat, jedoch nicht Wasserstoff bedeutet,

$R^8$ die für $R^5$ angegebene Bedeutung hat und mit diesem gleich oder verschieden sein kann,

$R^5$ für Aryl oder Heteroaryl steht, wobei die Aryle sowie die Heteroaryle gegebenenfalls 1 bis 3 gleiche oder verschiedene Substituenten aus der Gruppe Phenyl, Alkyl, Cycloalkyl, Morpholinyl, Alkoxy, Alkylen, Alkenyl, Alkenyloxy, Dioxyalkylen, Halogen, Polyfluoralkoxy, Nitro, Cyano, Azido, $SO_m-R^6$, $R^6R^7N-SO_m$ ($m = 0$ bis 2), $NR^6R^7$, $NR^6COR^7$ und Acyl der Formel $CO-R^6$ enthalten können, und wobei die Alkylreste, ihrerseits gegebenenfalls durch ein oder zwei Sauerstoffatome, Schwefelatome oder

Gruppierungen der Formel $-NR^6-$, $-CONR^7-$ oder $\underset{H}{N}CO_2-$ unterbrochen sein kann und/oder durch Halogen substituiert sein kann, und wobei $R^6$ und $R^7$ jeweils gleich oder verschieden sind und die obengenannte Bedeutung haben,

A für eine geradkettige, verzweigte oder cyclische Alkylengruppe mit bis zu 20 Kohlenstoffatomen steht, die durch eine gegebenenfalls durch Halogen, Cyano, Dialkylamino, Alkyl, Alkoxy, Trifluormethyl oder Nitro substituierte Phenylgruppe substituiert oder unterbrochen sein kann, und/oder die gegebenenfalls durch ein oder mehrere Sauerstoffatome, Schwefelatome oder Gruppierungen der Formel $-NR^6-$ oder $-CO-NR^7$ unterbrochen ist, wobei

$R^6$, $R^7$ die oben angegebene Bedeutung haben,

und

X für die Gruppe $-COR^9$ steht, worin $R^9$ gegebenenfalls substituiertes Alkyl, Aryl, Aralkyl oder Anilino oder eine Amino-, Monoalkylamino- oder Dialkylaminogruppe bedeutet, wobei gegebenenfalls die Alkylgruppen mit dem Stickstoffatom einen 5- bis 7-gliedrigen Ring bilden, der als weiteres Heteroatom ein Sauerstoff- oder Schwefelatom enthalten kann,

oder für die Gruppe $-COOR^{10}$ steht, worin $R^{10}$ die gleiche Bedeutung hat wie $R^1$ und mit diesem gleich oder verschieden sein kann,

sowie deren physiologisch unbedenklichen Säureadditionsprodukte.

2. Verbindungen der allgemeinen Formel (I) gemäß Anspruch 1, in welcher

R für Phenyl, Naphthyl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit bis zu 8 Kohlenstoffatomen, Cycloalkyl mit 3 bis 7 Kohlenstoffatomen, Alkoxy mit 1 bis 4 Kohlenstoffatomen, Tri-, Tetra- und Pentamethylen, Dioxymethylen, Halogen, Trifluormethyl, Trifluormethoxy, Difluormethoxy, Tetrafluorethoxy, Nitro, Cyano, Azido oder $SO_m$-Alkyl, worin m eine Zahl von 0 bis 2 bedeutet und Alkyl 1 bis 4 Kohlenstoffatomen enthält, substituiert sein können,

$R^1$ einen geradkettigen, verzweigten oder cyclischen, gesättigten oder ungesättigten Kohlenwasserstoffrest mit bis zu 16 C-Atomen bedeutet, der gegebenenfalls durch ein Sauerstoff- oder ein Schwefelatom in der Kette unterbrochen ist, und/oder der gegebenenfalls substituiert ist durch Fluor, Chlor, Brom, Cyano, Hydroxy, Pyridyl, Acyloxy mit bis zu 4 C-Atomen oder durch eine Phenyl-, Phenoxy-, Phenylthio- oder Phenylsulfonylgruppe, welche ihrerseits wieder durch Fluor, Chlor, Brom, Cyano, Dialkylamino (1 bis 4 C-Atome), Alkoxy (1 bis 4 C-Atome), Alkyl (1 bis 4 C-Atome), Trifluormethyl oder Nitro substituiert sein können, oder der gegebenenfalls durch eine Aminogruppe substituiert ist, wobei diese Aminogruppe zwei gleiche oder verschiedene Substituenten aus der Gruppe Alkyl (1 bis 4 C-Atome), Alkoxyalkyl (bis zu 8 C-Atome), Phenyl, Naphthyl oder Phenylalkyl mit 1 bis 4 C-Atomen im Alkylrest trägt und wobei diese Substituenten gegebenenfalls mit dem Stickstoffatom einen 5- bis 7-gliedrigen Ring bilden, der als weiteres Heteroatom ein Sauerstoff- oder Schwefelatom oder eine N-Alkyl-Gruppierung mit 1 bis 4 C-Atomen enthalten kann,

$R^2$ für Wasserstoff, Cyano, Phenyl oder Benzyl, oder für geradkettiges oder verzweigtes Alkyl mit bis zu 4 Kohlenstoffatomen steht, das gegebenenfalls substituiert ist durch OH, Acetoxy, Cyano, ein oder mehrere Fluor, Chlor oder Brom,

$R^3$ für Wasserstoff, für geradkettiges oder verzweigtes, gegebenenfalls durch ein Sauerstoffatom unterbrochenes Alkyl mit bis zu 6 Kohlenstoffatomen, für Phenyl oder Benzyl steht,

$R^4$ für Wasserstoff, für geradkettiges oder verzweigtes Alkyl mit bis zu 6 C-Atomen,
für Phenyl, Benzyl oder
für einen Rest der Formel $CO-R^6$, $CO_2R^{6'}$, $CO-NR^6R^7$,

$SO_2R^{6'}$, $-CH_2-CH(OH)-CH_2-OR^8$ steht,

wobei

$R^6$ und $R^7$ gleich oder verschieden sind und jeweils für Wasserstoff oder einen Kohlenwasserstoffrest von 1 bis 6 Kohlenstoffatomen, Phenyl oder Benzyl stehen,

$R^{6'}$ die Bedeutung von $R^6$ hat, jedoch nicht Wasserstoff bedeutet,

$R^8$ die für $R^5$ angegebene Bedeutung hat und mit diesem gleich oder verschieden sein kann

$R^5$ für Phenyl, Naphthyl oder für Furyl, Thienyl, Pyrryl, Pyrazolyl, Imidazolyl, Oxazolyl, Isoxazolyl, Thiazolyl, Thiadiazolyl, Pyridyl, Pyridazinyl, Pyrazinyl, Indolyl, Benzimidazolyl, Benzoxazolyl, Benzoxadiazolyl, Benzthiadiazolyl, Chinolyl, Isochinolyl, Chinazolyl, Chinoxazolyl oder Carbazolyl steht, wobei die

genannten Ringsysteme gegebenenfalls 1 bis 2 gleiche oder verschiedene Substituenten aus der Gruppe Phenyl, Alkyl (1 - 8 C-Atome), Alkenyl (2 - 8 C-Atome), Cycloalkyl (5 - 7 C-Atome), Alkoxy (1 - 4 C-Atome), Alkenyloxy (2 - 4 C-Atome), Alkylen (2 - 4 C-Atome), Morpholinyl, Dioxalkylen (1 - 3 C-Atome), Halogen, Polyfluoralkoxy (1 bis 4 C-Atome), Nitro, Cyano, Azido, $SO_m$-$R^6$, $R^6R^7N$-$SO_m$ (m = 0 oder 2), $NR^6R^7$, $NR^6COR^7$ und Acyl der Formel CO-$R^6$ enthalten können, wobei der Alkylsubstituent gegebenenfalls durch ein oder zwei Sauerstoffatome, Schwefelatome oder Gruppierungen der Formel -$NR^6$-, -$CONR^7$- oder -NCO- unterbrochen sein kann, und/oder durch Halogen
H
substituiert sein kann, und wobei $R^6$ und $R^7$ jeweils die obengenannte Bedeutung besitzen,

A für eine geradkettige, verzweigte oder cyclische Alkylengruppe mit bis zu 20 Kohlenstoffatomen steht, die gegebenenfalls durch eine Phenylgruppe, welche ihrerseits durch Halogen, Cyano, Dialkylamino, Alkyl, Alkoxy mit je 1 bis 4 C-Atomen, Trifluormethyl oder Nitro substituiert sein kann, substituiert oder unterbrochen ist, und/oder die gegebenenfalls durch ein oder zwei Sauerstoffatome, Schwefelatome und Gruppierungen der Formel -$NR^6$- oder -CO-$NR^7$- unterbrochen ist, wobei $R^6$, $R^7$ die oben angegebene Bedeutung haben, und

X für die Gruppe $-COR^9$ steht, worin $R^9$ gegebenenfalls durch Halogen, Cyano, Alkoxy oder Dialkylamino mit jeweils 1 bis 4 C-Atomen substituiertes Alkyl mit 1 bis 7 C-Atomen, Phenyl, Naphthyl, Phenylalkyl oder Anilino oder eine Amino-, Monoalkylamino- oder Dialkylaminogruppe mit je 1 bis 4 C-Atomen bedeutet, wobei gegebenenfalls die Alkylgruppen mit dem Stickstoffatom einen 5- bis 7-gliedrigen Ring bilden, der als weiteres Heteroatom ein Sauerstoff- oder Schwefelatom enthalten kann,

oder für die Gruppe $-COOR^{10}$ steht, wobei $R^{10}$ die gleiche Bedeutung hat wie $R^1$ und mit diesem gleich oder verschieden sein kann.

3. Verbindungen der allgemeinen Formel (I), in welcher

R für Phenyl, Thienyl, Furyl, Pyridyl oder Benzoxadiazolyl steht, wobei der Phenylrest 1 oder 2 gleiche oder verschiedene Substituenten aus der Gruppe Nitro, Trifluormethyl, Fluor, Chlor, Cyano, Alkoxy mit bis zu 4 C-Atomen oder Dioxymethylen enthält,

$R^1$ einen geradkettigen, verzweigten oder cyclischen Alkylrest mit bis zu 12 C-Atomen steht, der gegebenenfalls durch ein Sauerstoffatom in der Kette unterbrochen ist und/oder der gegebenenfalls substituiert ist durch Fluor, Chlor, Hydroxy, Pyridyl, Phenyl oder Amino,

$R^2$ für Phenyl, Benzyl, Methyl, Ethyl, Acetoxymethyl, Trifluormethyl, Chlormethyl oder Brommethyl steht,

$R^3$ für Wasserstoff oder Alkyl mit 1 bis 2 Kohlenstoffatomen steht,

$R^4$ für Wasserstoff,
für geradkettiges oder verzweigtes Alkyl mit bis zu 4 C-Atomen, für Benzyl oder
für einen Rest der Formel $-CO-R^6$, $-CO_2R^{6'}$, $CO-NR^6R^7$, $SO_2R^{6'}$, $-CH_2-CH(OH)-CH_2-OR^8$ steht,

wobei

$R^6$ und $R^7$ gleich oder verschieden sind und jeweils für Wasserstoff, Alkyl mit bis zu 4 C-Atomen, Phenyl oder Benzyl steht,

$R^{6'}$ die Bedeutung von $R^6$ hat, jedoch nicht Wasserstoff bedeutet,

$R^8$ die für $R^5$ angegebene Bedeutung hat und mit diesem gleich oder verschieden sein kann,

$R^5$ für Phenyl, Naphthyl oder Indolyl steht, wobei der Phenylrest gegebenenfalls durch 1 oder 2 gleiche oder verschiedene Substituenten aus der Gruppe Fluor, Chlor, Nitro, Cyano, Cyclopentyl, Cyclohexyl, Alkyl mit 1 bis 2 C-Atomen, Alkoxy mit 1 bis 2 C-Atomen, Allyl, Allyloxy, $NR^6R^7$, $NR^6COR^7$ oder Acetyl substituiert ist,

A für eine geradkettige, verzweigte oder cyclische Alkylengruppe mit bis zu 12 Kohlenstoffatomen steht, die gegebenenfalls durch eine Phenylgruppe substituiert oder unterbrochen ist und/oder die gegebenenfalls durch ein Sauerstoffatom oder eine Gruppierung der Formel $-NR^6-$ oder $-CONR^7-$ unterbrochen ist, wobei $R^6$, $R^7$ die angegebene Bedeutung haben,

und

X für die Gruppe $-COOR^{10}$ steht, wobei $R^{10}$ die gleiche Bedeutung hat wie $R^1$ und mit diesem gleich oder verschieden sein kann.

4. Verfahren zur Herstellung von Verbindungen der allgemeinen Formel (I)

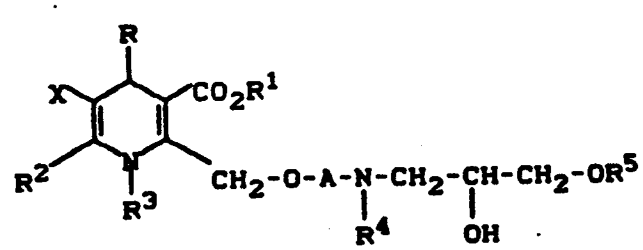

(I)

in welcher

R für Aryl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei der Arylrest sowie die Heterocyclen gegebenenfalls 1 bis 2 gleiche oder verschiedene Substituenten aus

der Gruppe Phenyl, Alkyl, Alkoxy, Alkylen, Dioxyalkylen, Halogen, Trifluormethyl, Polyfluoralkoxy, Nitro, Cyano, Azido oder $SO_m$-Alkyl (m = 0 bis 3) enthalten,

$R^1$ einen geradkettigen, verzweigten oder cyclischen, gesättigten oder ungesättigten Kohlenwasserstoffrest bedeutet, der gegebenenfalls durch ein Sauerstoff- oder ein Schwefelatom in der Kette unterbrochen ist, und/oder der gegebenenfalls substituiert ist durch Halogen, Cyan, Hydroxy, Acyloxy, Pyridyl oder durch eine Phenyl-, Phenoxy-, Phenylthio- oder Phenylsulfonylgruppe, wobei die Arylreste ihrerseits wieder durch Halogen, Cyano, Dialkylamino, Alkoxy, Alkyl, Trifluormethyl oder Nitro subtituiert sein können,

oder der gegebenenfalls durch eine Aminogruppe substituiert ist, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem Stickstoffatom einen 5- bis 7-gliedrigen Ring bilden, der als weiteres Heteroatom ein Sauerstoff- oder Schwefelatom oder eine N-Alkyl-Gruppierung enthalten kann,

$R^2$ für Wasserstoff, Cyano, Aryl, Aralkyl oder für geradkettiges oder verzweigtes Alkyl steht, das gegebenenfalls substituiert ist durch Cyano, OH, Acetoxy oder Halogen,

$R^3$ für Wasserstoff oder geradkettiges oder verzweigtes Alkyl steht, das gegebenenfalls durch ein oder zwei Sauerstoffatome in der Alkylkette unterbrochen ist, oder für Aryl oder Aralkyl steht,

$R^4$ für Wasserstoff,
für geradkettiges oder verzweigtes Alkyl,
für Aryl, Aralkyl oder
für einen Rest der Formel $CO\text{-}R^6$, $CO_2\text{-}R^{6'}$,
$CO\text{-}NR^6R^7$, $SO_2R^6$, $-CH_2-\underset{\displaystyle OH}{\underset{|}{C}H}-CH_2-OR^8$ steht,

wobei

$R^6$ und $R^7$ gleich oder verschieden sind und jeweils für Wasserstoff, einen niedrigen Kohlenwasserstoffrest, Aryl oder Aralkyl stehen,

$R^{6'}$ die Bedeutung von $R^6$ hat, jedoch nicht Wasserstoff bedeutet,

$R^8$ die für $R^5$ angegebene Bedeutung hat und mit diesem gleich oder verschieden sein kann,

$R^5$ für Aryl oder Heteroaryl steht, wobei die Aryle sowie die Heteroaryle gegebenenfalls 1 bis 3 gleiche oder verschiedene Substituenten aus der Gruppe Phenyl, Alkyl, Cycloalkyl, Morpholinyl, Alkoxy, Alkylen, Alkenyl, Alkenyloxy, Dioxyalkylen, Halogen, Polyfluoralkoxy, Nitro, Cyano, Azido, $SO_m\text{-}R^6$, $R^6R^7N\text{-}SO_m$ (m = 0 bis 2), $NR^6R^7$, $NR^6COR^7$ und Acyl der Formel $CO\text{-}R^6$ enthalten können, und wobei die Alkylreste, ihrerseits gegebenenfalls durch ein oder zwei Sauerstoffatome, Schwefelatome oder

Gruppierungen der Formel $-NR^6-$, $-CONR^7-$ oder $\underset{H}{N}CO_2-$ unterbrochen sein kann und/oder durch Halogen substituiert sein kann, und wobei $R^6$ und $R^7$ jeweils gleich oder verschieden sind und die obengenannte Bedeutung haben,

A für eine geradkettige, verzweigte oder cyclische Alkylengruppe mit bis zu 20 Kohlenstoffatomen steht, die durch eine gegebenenfalls durch Halogen, Cyano, Dialkylamino, Alkyl, Alkoxy, Trifluormethyl oder Nitro substituierte Phenylgruppe substituiert oder unterbrochen sein kann, und/oder die gegebenenfalls durch ein oder mehrere Sauerstoffatome, Schwefelatome oder Gruppierungen der Formel $-NR^6-$ oder $-CO-NR^7$ unterbrochen ist, wobei

$R^6$, $R^7$ die oben angegebene Bedeutung haben,

und

X für die Gruppe $-COR^9$ steht, worin $R^9$ gegebenenfalls substituiertes Alkyl, Aryl, Aralkyl oder Anilino oder eine Amino-, Monoalkylamino- oder Dialkylaminogruppe bedeutet, wobei gegebenenfalls die Alkylgruppen mit dem Stickstoffatom einen 5- bis 7-gliedrigen Ring bilden, der als weiteres Heteroatom ein Sauerstoff- oder Schwefelatom enthalten kann,

oder für die Gruppe $-COOR^{10}$ steht, worin $R^{10}$ die gleiche Bedeutung hat wie $R^1$ und mit diesem gleich oder verschieden sein kann,

sowie deren physiologisch unbedenklichen Säureadditionsprodukte,

dadurch gekennzeichnet, daß man Dihydropyridine der allgemeinen Formel (II)

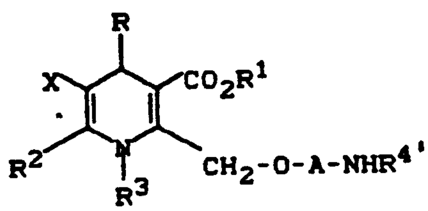

(II)

in welcher

$R$, $R^1$, $R^2$, $R^3$, X und A die oben angegebene Bedeutung haben, und

$R^{4'}$ für Wasserstoff, Alkyl, Aryl oder Aralkyl steht,

mit Epoxiden der allgemeinen Formel (III)

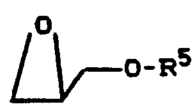

(III)

in welcher

$R^5$ die oben angegebene Bedeutung hat,

gegebenenfalls in Gegenwart inerter organischer Lösungsmittel umsetzt.

5. Verfahren zur Herstellung von Verbindungen der allgemeinen Formel (I), in welcher

$R^4$ für den Rest $CH_2-\underset{OH}{CH}-CH_2-OR^8$ steht, wobei $R^8$ von $R^5$ verschieden ist,

gekennzeichnet durch stufenweise Umsetzung von primären Dihydropyridinaminen (II) mit zunächst einem Epoxid mit dem Substituenten $R^5$ (III) und anschließend einem weiteren Epoxid mit dem Substituenten $R^8$ der allgemeinen Formel IIIa

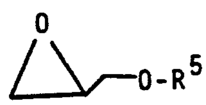 und 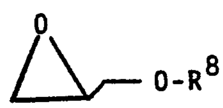 .

(III) (IIIa)

6. Verfahren zur Herstellung von Verbindungen der allgemeinen Formel (I) gemäß Anspruch 4, in welcher $R^4$ für die Reste $CO-R^8$, $CO_2R^6$, $CO-NR^6R^7$ oder $SO_2R^6$ steht, dadurch gekennzeichnet, daß man Verbindungen der Formel (I), in welcher $R^4$ Wasserstoff bedeutet, mit Acylierungs- oder Sulfonylierungsmitteln umsetzt.

7. Verbindungen der allgemeinen Formel (IIa)

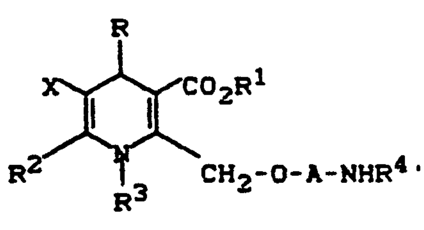 (IIa),

in welcher

R für Aryl oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Chinolyl, Isochinolyl, Chinazolyl oder Chinoxalyl steht, wobei der Arylrest sowie die Heterocyclen gegebenenfalls 1 bis 2 gleiche oder verschiedene Substituenten aus der Gruppe Phenyl, Alkyl, Alkoxy, Alkylen, Dioxyalkylen, Halogen, Trifluormethyl, Polyfluoralkoxy, Nitro, Cyano, Azido oder $SO_m$-Alkyl (m = 0 bis 3) enthalten,

$R^1$ einen geradkettigen, verzweigten oder cyclischen, gesättigten oder ungesättigten Kohlenwasserstoffrest bedeutet, der gegebenenfalls durch ein Sauerstoff- oder ein Schwefelatom in der Kette unterbrochen ist, und/oder der gegebenenfalls substituiert ist durch Halogen, Cyan, Hydroxy, Acyloxy, Pyridyl oder durch ein Phenyl-, Phenoxy-, Phenylthio- oder Phenylsulfonylgruppe, wobei die Arylreste ihrerseits wieder durch Halogen, Cyano, Dialkylamino, Alkoxy, Alkyl, Trifluormethyl oder Nitro substituiert sein können,

oder der gegebenenfalls durch eine Aminogruppe substituiert ist, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem

Stickstoffatom einen 5- bis 7-gliedrigen Ring bilden, der als weiteres Heteroatom ein Sauerstoff- oder Schwefelatom oder eine N-Alkyl-Gruppierung enthalten kann,

$R^2$ für Wasserstoff, Cyano, Aryl, Aralkyl oder für geradkettiges oder verzweigtes Alkyl steht, das gegebenenfalls substituiert ist durch Cyano, OH, Acetoxy oder Halogen,

$R^3$ für Wasserstoff oder geradkettiges oder verzweigtes Alkyl steht, das gegebenenfalls durch ein oder zwei Sauerstoffatome in der Alkylkette unterbrochen ist, oder für Aryl oder Aralkyl steht,

$R^4$ für Wasserstoff, Alkyl, Aryl oder Aralkyl steht,

A für eine geradkettige, verzweigte oder cyclische Alkylengruppe mit bis zu 20 Kohlenstoffatomen steht, die durch eine gegebenenfalls durch Halogen, Cyano, Dialkylamino, Alkyl, Alkoxy, Trifluormethyl oder Nitro substituierte Phenylgruppe substituiert oder unterbrochen sein kann, und/oder die gegebenenfalls durch ein oder mehrere Sauerstoffatome, Schwefelatome oder Gruppierungen der Formel $-NR^6-$ oder $-CO-NR^7-$ unterbrochen ist, wobei

$R^6$ und $R^7$ gleich oder verschieden sind und jeweils für Wasserstoff, einen niedrigen Kohlenwasserstoffrest, Aryl oder Aralkyl stehen, und

wobei

A jedoch nicht für die Gruppen $-(CH_2)_2$, $-(CH_2)_3-$, $-CH_2CH(CH_3)-$ und $-CH_2C(CH_3)_2-$ stehen darf,

und

X für die Gruppe $-COR^9$ steht, worin $R^9$ gegebenenfalls substituiertes Alkyl, Aryl, Aralkyl oder Anilino oder eine Amino-, Monoalkylamino- oder Dialkylaminogruppe bedeutet, wobei gegebenenfalls die Alkylgruppen mit dem Stickstoffatom einen 5- bis 7-gliedrigen Ring bilden, der als weiteres Heteroatom ein Sauerstoff- oder Schwefelatom enthalten kann,

oder für die Gruppe $-COOR^{10}$ steht, worin $R^{10}$ die gleiche Bedeutung hat wie $R^I$ und mit diesem gleich oder verschieden sein kann,

sowie deren Säureadditionsprodukte.

8. Verfahren zur Herstellung von Verbindungen der allgemeinen Formel (IIa) gemäß Anspruch 7, dadurch gekennzeichnet, daß man Verbindungen der allgemeinen Formel (IV)

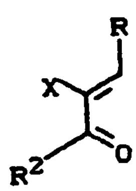

(IV),

in welcher

R, $R^2$ und X die in Anspruch 7 angegebene Bedeutung haben, mit Enaminen der allgemeinen Formel (V)

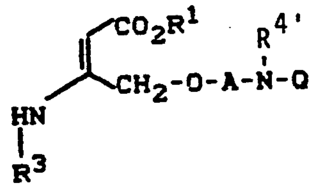 (V),

in welcher $R^1$, $R^3$, $R^{4'}$ und A die in Anspruch 7 angegebene Bedeutung haben, und

Q für eine Aminoschutzgruppe, bevorzugt für tert. Butyloxycarbonyl oder Benzyl steht oder
$R^{4'}$ und Q gemeinsam für Phthalimid stehen,

in inerten Lösungsmitteln umsetzt und anschließend in bekannter Weise die Aminoschutzgruppe abspaltet.

9. Arzneimittel, enthaltend mindestens eine Verbindung der allgemeinen Formel (I) gemäß Anspruch 1.

10. Verbindungen der allgemeinen Formel (I) gemäß Anspruch 1 zur Verwendung bei der Bekämpfung von Erkrankungen.

11. Verwendung von Verbindungen der allgemeinen Formel (I) gemäß Anspruch 1 bei der Herstellung von kreislaufwirksamen Mitteln.

12. Verwendung von Verbindungen der allgemeinen Formel (IIa) gemäß Anspruch 7 zur Herstellung von Verbindungen der allgemeinen Formel (I) gemäß Anspruch 1.

0218068

Europäisches Patentamt

# EUROPÄISCHER RECHERCHENBERICHT

Nummer der Anmeldung

EP 86 11 1731

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 151 006 (YAMANOUCHI PHARMACEUTICAL CO. LTD.)<br>* Seite 2; Ansprüche 1, 4-6, 9 * | 1-4,9-12 | C 07 D 211/90<br>C 07 D 413/04<br>C 07 D 401/12<br>C 07 D 401/04<br>C 07 D 405/04<br>C 07 D 405/12<br>C 07 D 409/04<br>C 07 D 409/12<br>C 07 D 413/12<br>C 07 D 417/04<br>C 07 D 417/12<br>C 07 D 401/14<br>C 07 D 405/14<br>C 07 D 409/14<br>C 07 D 413/14<br>C 07 D 417/14<br>A 61 K 31/44<br>C 07 B 53/00 |
| Y | EP-A-0 060 674 (PFIZER LTD., PFIZER CORP.)<br>* Ansprüche 1-3, 5, 6 * | 1-4,9-12 | |
| Y | DE-B-2 407 115 (YAMANOUCHI PHARMACEUTICAL CO. LTD.)<br>* Ansprüche 1, 3 * | 1-4,9-12 | |
| A,P | PATENT ABSTRACTS OF JAPAN, Band 9, Nr. 293 (C-315)[2016], 20. November 1985; & JP - A - 60 136 558 (TEIKOKU ZOKI SEIYAKU K.K.) 20.07.1985 | 1-4,9-12 | **RECHERCHIERTE SACHGEBIETE (Int. Cl.4)** |
| A,D | EP-A-0 089 167 (PFIZER LTD., PFIZER CORP.)<br>* Seiten 2-10 * | 7,8 | C 07 D 211/00<br>C 07 D 413/00<br>C 07 D 401/00<br>A 61 K 31/00<br>C 07 D 405/00<br>C 07 D 409/00<br>C 07 D 417/00 |
| A | EP-A-0 116 769 (PFIZER LTD., PFIZER CORP.)<br>* Seite 10, Zeile 13 - Seite 12, Zeile 3; Seite 3, Zeilen 5-9 * | 7, 8 | |

Der vorliegende Recherchenbericht wurde für alle Patentansprüche erstellt.

| Recherchenort | Abschlußdatum der Recherche | Prüfer |
|---|---|---|
| BERLIN | 04-12-1986 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82